# EUROPEAN PATENT SPECIFICATION

(11) **EP 1 616 195 B1**
(45) Date of publication and mention of the grant of the patent: **07.11.2007**
(21) Application number: 04728206.6
(22) Date of filing: 19.04.2004
(51) Int. Cl.: G01N 33/68

(54) **SCREEN FOR PRE-ECLAMPSIA**
SCREEN FÜR PRÄEKLAMPSIE
ASSAI DESTINE A LA PRE-ECLAMPSIE

(30) Priority: 17.04.2003 GB 0308967
(43) Date of publication of application: 18.01.2006
(73) Proprietor: UNIVERSITY COLLEGE LONDON, London WC1E 9BT (GB)
(72) Inventor: SAVVIDOU, Makrina, Denmark Hill, London SE5 8RS (GB); HINGORANI, Aroon, Centre For Clinical Pharmacology, London WC1E 6JF (GB); VALLANCE, P., Centre For Clinical Pharmacology, London WC1E 6JF (GB); NICOLAIDES, Kypros, King's College Hospital, London SE5 8RS (GB)
(74) Representative: Woods, Geoffrey Corlett
(86) International application number: PCT/GB2004/001701
(87) International publication number: WO 2004/095032

(56) References cited:
- WO-A-02/14873
- WO-A-20/04046314
- DE-A- 10 040 904
- US-A- 5 811 416
- ELLIS J ET AL: "Levels of dimethylarginines and cytokines in mild and severe preeclampsia." ACTA OBSTETRICIA ET GYNECOLOGICA SCANDINAVICA. JUL 2001, vol. 80, no. 7, July 2001 (2001-07), pages 602-608, XP002287424 ISSN: 0001-6349
- PETTERSSON ANDERS ET AL: "Increased circulating concentrations of asymmetric dimethyl arginine (ADMA), an endogenous inhibitor of nitric oxide synthesis, in preeclampsia" ACTA OBSTETRICIA ET GYNECOLOGICA SCANDINAVICA, vol. 77, no. 8, September 1998 (1998-09), pages 808-813, XP002287425 ISSN: 0001-6349
- DAYOUB H ET AL: "Lessons from a DDAH transgenic mouse: Role of ADMA in regulating NOS activity and blood pressure." EUROPEAN HEART JOURNAL, vol. 23, no. Abstract Supplement, 4 September 2002 (2002-09-04), page 132, XP008032589 & CONGRESS OF THE EUROPEAN SOCIETY OF CARDIOLOGY; BERLIN, GERMANY; AUGUST 31-SEPTEMBER 04, 2002 ISSN: 0195-668X
- SAVVIDOU M D ET AL: "Endothelial dysfunction and raised plasma concentrations of asymmetric dimethylarginine in pregnant women who subsequently develop pre-eclampsia" LANCET THE, LANCET LIMITED. LONDON, GB, vol. 361, no. 9368, 3 May 2003 (2003-05-03), pages 1511-1517, XP004423226 ISSN: 0140-6736
- DAYOUB HAYAN ET AL: "Dimethylarginine dimethylaminohydrolase regulates nitric oxide synthesis: Genetic and physiological evidence." CIRCULATION, vol. 108, no. 24, 16 December 2003 (2003-12-16), pages 3042-3047, XP008032592 ISSN: 0009-7322
- SAVVIDOU MD ET AL.: "Levels of C-reactive protein in pregnant women who subsequently develop pre-eclampsia" BJOG, vol. 109, March 2002 (2002-03), pages 297-301,
- DATABASE PUBMED Obstetrics and Gynecology vol 77 nr 2 p 176-178 1991, WATTS DH ET AL.: "C-REACTIVE PROTEIN IN NORMAL PREGNANCY"
- PEARSON TA ET AL.: "Markers of Inflammation and Cardiovascular Disease: Application to Clinical and Public Health Practice: A statement for healthcare professionals from the Centers for Disease Control and Prevention and the American Heart Association." CIRCULATION, vol. 107, no. 3, January 2003 (2003-01), pages 499-511,

## Description

### Field of the invention

The invention relates to the diagnosis of susceptibility to and the prevention of the development of pre-eclampsia and intrauterine growth restriction (IUGR).

### Background of the invention

Pre-eclampsia is a common disorder affecting 3 to 5% of human pregnancies and accounting for 40% of iatrogenic deliveries in the UK. It is part of a group of hypertensive disorders that also includes eclampsia, latent chronic essential hypertension, renal diseases and transient gestational hypertension. The disorder is typically defined as acute hypertension accompanied by abnormal proteinuria developing after 20 weeks geststaion in a previously normotensive woman (Davey DA et al. Am J Obstet Gynecol 1988; 158: 892-8). It is also typically associated with pulmonary oedema, cyanosis, impaired liver function, visual or cerebral disturbances, pain in the epigastric area or right upper quadrant, decreased platelet count, intrauterine-growth restriction (IUGR) or oliguria. At present, the pathogenesis of pre-eclampsia is poorly understood, limiting the development of a reliable predictive test or effective prophylaxis.

### Summary of the invention

Asymmetric dimethylarginine (ADMA) is an endogenous inhibitor of nitric oxide synthase (NOS) that competes with binding of the natural substrate L-arginine. It is produced from methylated arginine residues in proteins by protein methyltransferases (PRMT) and is metabolised by the enzyme dimethylarginine dlmethylaminohydrolase (DDAH). ADMA is produced in the fetoplacental unit, which contains large amounts of DDAH II. The inventors have shown for the first time that levels of ADMA are increased in women that subsequently develop pre-eclampsia and whose children develop IUGR.

According to the invention there is thus provided a method of identifying whether or not a pregnant woman is at risk of developing pre-eclampsia or whether or not her fetus is at risk of developing intrauterine growth restriction (IUGR), which method comprises:
a) measuring ADMA in a fluid sample taken from a pregnant woman at a stage of pregnancy from 4 to 25 weeks gestation; and
b) determining whether or not the ADMA is greater than 2.0µmol/L in the fluid sample, thereby determining whether or not the woman is at risk of developing pre-eclampsia or her fetus is at risk of developing IUGR.

### Description of the Figures

Figure 1 shows flow-mediated dilatation (FMD) of the brachial artery at 23 to 25 weeks of gestation at the different group of women depending on the presence or absence of bilateral notches and on the outcome of pregnancy. The horizontal lines illustrate the mean FMD in each group.
Figure 2 shows plasma levels of ADMA in the two groups of pregnant women (with and without bilateral notches in the uterine artery Doppler examination). The horizontal lines illustrate the median ADMA levels in each group.
Figure 3 shows scatterplot illustrating the relationship between flow-mediated dilatation and plasma levels of ADMA in the group of pregnant women who had bilateral notches of the uterine arteries at 23 to 25 weeks of gestation and eventually developed pre-eclampsia. A significant inverse correlation was found (r= - 0.8, p=0.005).

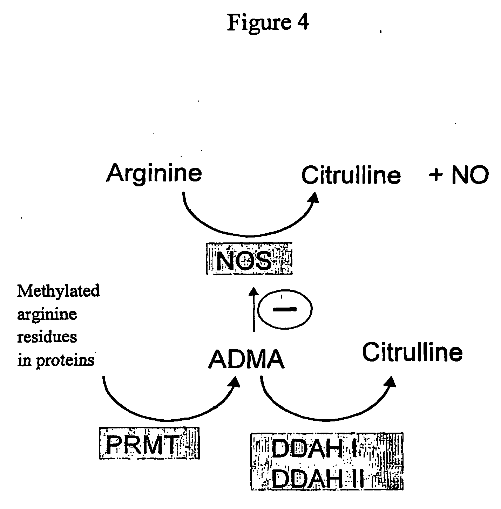

Figure 4 shows synthesis of ADMA from methylated arginine residues in proteins by protein methylarginases (PRMT), and its metabolism to citrulline through the action of the dimethylarginine dimethyldiaminohydrolases I and II (DDAH I and II).

### Detailed description of the invention

The present invention relates to a method of identifying whether or not a pregnant woman is at risk of developing pre-eclampsia or whether or not her fetus is at risk of developing IUGR The invention therefore relates to the diagnosis of susceptibility of a pregnant woman to pre-eclampsia and the diagnosis of susceptibility of a fetus to IUGR. The pregnant woman is a human being. The fetus is human. The woman or fetus who is diagnosed may be in the first, second or third trimester of pregnancy. The woman or fetus is at a stage of pregnancy from 4 to 25 weeks gestation. The woman or fetus may be at a stage of pregnancy from 23 to 25 weeks gestation. Preferably the woman or fetus is at a stage of pregnancy from 10 to 25 weeks gestation and more preferably from 15 to 25 weeks gestation.

Typically the woman does not have pre-eclampsia or displays no symptoms of pre-eclampsia. However, the method of the invention may be carried out on women who have pre-eclampsia but have not been tested for it. The method may therefore be carried out on women who display preliminary symptoms of pre-eclampsia.

Typically the fetus does not have IUGR or displays no symptoms of IUGR. However, the method of the invention may be carried out on women whose fetuses have IUGR but have not been tested for it. The method may therefore be carried out on women whose fetuses display preliminary symptoms of IUGR.

The present invention involves measuring ADMA in the woman. Typically the level or concentration of ADMA is measured. According to the present invention, an increased level of ADMA compared with the normal pregnancy level indicates that the woman is susceptible to or at risk of developing pre-eclampsia or her fetus is susceptible to or at risk of developing IUGR. The normal pregnancy level is typically the level of ADMA in a woman who displays no symptoms of pre-eclampsia or whose fetus does not display symptoms of IUGR throughout the entire pregnancy. The normal pregnancy level is typically at an equivalent stage of pregnancy.

The mean plasma ADMA concentration in a normal non-pregnant population is typically about 0.82µmol/L. Generally the mean plasma ADMA concentration in a normal pregnant human is lower than that in a normal non-pregnant individual and remains relatively constant throughout pregnancy. The normal pregnancy level for a human at 4, 10, 15 and 25 weeks of pregnancy is typically about 0.3 to 0.6µmol/L, for example 0.52µmol/L in plasma.

In the present invention, an increased plasma level of ADMA associated with increased susceptibility to or risk of developing of pre-eclampsia or increased susceptibility to or risk of developing of IUGR is greater than about 2.0µmol/L. According to the present invention, the ADMA level/concentration in the woman is preferably increased by at least 4 fold compared to the normal pregnancy level. The ADMA, level/concentration is typically raised by 3 to 7 fold compared to the normal pregnancy level.

Symmetric dimethylarginine (SDMA) is the biologically inactive stereosiomer of ADMA. SDMA is not metabolized by DDAH but is instead excreted by the kidney. The L-arginine/ADMA ratio is typically used as an index of NOS inhibition. The ADMA/SDMA ratio typically reflects DDAH activity. The present invention may also involve assessment of the ADMA/SDMA ratio to determine the risk of developing pre-eclampsia.

According to the present invention, an increased ADMA/SDMA ratio compared with the normal pregnancy ratio indicates that the woman is susceptible to or at risk of developing pre-eclampsia or her fetus is susceptible to or at risk of developing IUGR. The normal pregnancy ratio is the ratio of ADMA/SDMA in a woman who displays no symptoms of pre-eclampsia or whose fetus displays no symptoms of IUGR throughout the entire pregnancy. The normal ADMA/SDMA ratio is typically at an equivalent stage of pregnancy.

The normal pregnancy ratio for a human at 4,10, 15 and 25 weeks of pregnancy is typically about 1:1 to 1.3:1, for example 1.3:1 in plasma. In the present invention, an increased plasma ratio of ADMA/SDMA associated with increased susceptibility to or risk of developing of pre-eclampsia or IUGR at 23 to 25 weeks is typically about 6.8:1. According to the present invention, the ADMA/SDMA ratio in the woman is preferably increased by at least 5 fold and more preferably by at least 6 fold compared to the normal pregnancy level, for example at the same stage of pregnancy. The ADMA/SDMA ratio is typically increased by 5 to 8 fold compared to the normal pregnancy level. According to the present invention, an ADMA/SDMA ratio above the 95% confidence interval for the normal pregnancy ratio is associated with an increased risk of developing pre-eclampsia or IUGR.

The invention is carried out *in vitro* on a sample obtained from the woman. The sample typically comprises a body fluid of the woman. The sample is preferably a blood, plasma, serum or urine sample but may be amniotic fluid. The sample is typically processed prior to being assayed, for example by centrifugation. The sample may also be typically stored prior to assay, preferably below -70°C.

Standard methods known in the art may be used to assay the level of ADMA. These methods typically involve contacting the sample with an antibody capable of binding to ADMA. Such methods include dipstick assays and Enzyme-linked Immunosorbant Assay (ELISA). Typically dipsticks comprise one or more antibodies or proteins that specifically bind ADMA. If more than one antibody is present, the antibodies preferably have different non-overlapping determinants such that they may bind to ADMA simultaneously.

ELISA is a heterogeneous, solid phase assay that requires the separation of reagents. ELISA is typically carried out using the sandwich technique or the competitive technique. The sandwich technique requires two antibodies. The first specifically binds ADMA and is bound to a solid support. The second antibody is bound to a marker, typically an enzyme conjugate. A substrate for the enzyme is used to quantify the ADMA-antibody complex and hence the amount of ADMA in a sample. The antigen competitive inhibition assay also typically requires an ADMA-specific antibody bound to a support. An ADMA-enzyme conjugate is added to the sample (containing ADMA) to be assayed. Competitive inhibition between the ADMA-enzyme conjugate and unlabeled ADMA allows quantification of the amount of ADMA in a sample. The solid supports for ELISA reactions preferably contain wells.

The present invention may also employ methods of measuring ADMA that do not comprise antibodies. High Performance Liquid Chromatography (HPLC) separation and fluorescence detection is preferably used as a method of determining the ADMA level. HPLC apparatus and methods as described previously may be used (Tsikas D et al. J Chromatogr B Biomed Sci Appl 1998; 705: 174-6) Separation during HPLC is typically carried out on the basis of size or charge. Prior to HPLC, endogenous amino acids and an internal standard L-homoarginine are typically added to assay samples and these are phase extracted on CBA cartridges (Varian, Harbor City, CA). Amino acids within the samples are preferably derivatized with o-phthalaldehyde (OPA). The accuracy and precision of the assay is preferably determined within quality control samples for all amino acids.

The present invention may be used to confirm susceptibility in women already suspected as being at risk or selected as being predisposed to developing pre-eclampsia. The present invention may also be used to confirm susceptibility in fetuses already suspected as being at risk or selected as being predisposed to developing IUGR Risk factors that increase susceptibility to developing pre-eclapmsia or IUGR typically include Afro-Caribbean ancestry, nullparity or first pregnancy with a partner, multiple gestations, hypertension, diabetes, genetic predisposition to or family history of pre-eclampsia or eclampsia, obesity, hypercholesterolaemia and smoking. The present invention may be used to determine the susceptibility of developing pre-eclampsia in smokers. The present invention may also be used to determine the susceptibility of the fetus of a smoker developing IUGR.

Some embodiments of the invention include additional diagnostic tests to determine susceptibility to pre-eclampsia of IUGR. Flow-mediated dilation of the branchial artery and/or Doppler waveform analysis of the uterine arteries are preferably employed. These diagnostic tests are typically carried out before, at the same time as or after measurement of the ADMA level in a pregnant woman.

Flow-mediated dilatation (FMD) of the brachial artery is an established non-invasive method of assessing endothelium-dependent vasodilation. It typically involves measuring changes in brachial artery diameter in response to increased flow using high resolution ultrasound. Ultrasonic apparatus and methods previously described may be used (Savvidou MD et al. Ultrasound Obstet Gynecol 2000; 15: 502-7; Dorup I et al. Am J Physiol 1999; 276: H821-5). The apparatus typically includes a linear array transducer. End-diastolic images of the artery may be stored in digital format. Arterial diameter is typically determined using a semi-automated edge detection algorithm. Baseline vessel diameter is typically calculated as the mean of all the measurements during the first minute of recording. FMD of the brachial artery is defined as the percentage increase in vessel diameter during reactive hyperaemia induced by inflation of a cuff distal to the site of the recording to 300 mmHg for 5 minutes followed by rapid deflation. Flow change (reactive hyperemia), an index of the flow stimulus for dilation, is typically calculated as [(blood flow 15 sec after cuff deflation-baseline blood flow)/baseline blood flow] X 100%. Endothelium-independent dilatation to sublingual glyceryl trinitrate (GTN) may be used as a control.

FMD of the brachial artery is typically 8.59 ± 2.76% (n=43) in normal pregnant human women. According to the invention, a significant reduction of FMD in addition to an increased AMDA level is indicative of a susceptibility to or a risk of developing pre-eclampsia The FMD of the brachial artery is preferably reduced by two fold or more preferably by at least two fold.

Doppler analysis is a routine method used to assess the waveform of the uterine arteries. The method is typically used to identify the presence or absence of early diastolic notches in the artery waveform. These notches may be unilateral or bilateral. According to the invention, the presence of a unilateral notch or bilateral notches in addition to an increased AMDA level is indicative of a susceptibility to pre-eclampsia or IUGR.

The diagnostic method of the invention may be carried out in conjunction with other assays or genetic tests to refine risk prediction.

The following Example illustrates the invention:

### Example

### 1. Methods

### Study Participants

All women attending for routine antenatal care at King's College Hospital had colour Doppler examination of their uterine arteries at 23-25 weeks of gestation (Acuson Aspen, California, USA). The Doppler waveforms of the uterine arteries were obtained as previously described (Albaiges G et al. Obstet Gynecol 2000; 96: 559-64.). When three similar consecutive waveforms were acquired the presence of an early diastolic notch was noted, and the mean pulsatility index (PI) of the two vessels was calculated. 43 pregnant women with abnormal uterine artery Doppler waveforms (presence of early diastolic notch bilaterally) identified consecutively, were recruited at the time of the Doppler study, and matched for age, ethnic group and smoking status with 43 pregnant women with normal uterine artery Doppler waveforms. At entry, all women had singleton pregnancies, were healthy, on no medications, had no personal or family history of premature cardiovascular disease, and had appropriately grown fetuses for the gestation. Maternal age, ethnic group, smoking status, parity, heart rate and BP were recorded. BP was measured in the right arm with the subject seated using an ambulatory blood pressure monitor (SpaceLabs Medical 90207, WA, USA). Three measurements were taken and averaged. The study was approved by the Local Ethics Committee and all subjects gave written informed consent

### Assessment of Maternal Endothelial Function

Ultrasound of the right brachial artery was performed using a 7 MHz linear array transducer and an Aspen Acuson system (California, USA) as previously described, at 23-25 weeks' gestation (Celermajer DS et al. Lancet 1992; 340: 1111-5; Savvidou MD et al. Obstet Gynecol 2000; 15: 502-7) End-diastolic images of the artery were acquired every 3 seconds and stored in digital format. Arterial diameter was determined for each image using a semi-automated edge detection algorithm. Baseline vessel diameter was calculated as the mean of all the measurements during the first minute of recording. FMD of the brachial artery was defined as the percentage increase in vessel diameter during reactive hyperaemia induced by inflation of a cuff distal to the site of the recording to 300 mmHg for 5 minutes followed by rapid deflation. Flow change (reactive hyperemia), an index of the flow stimulus for dilation, was calculated as [(blood flow 15 sec after cuff deflation-baseline blood flow)/baseline blood flow] X 100%. All the measurements were performed by an experienced operator and the FMD data analysed within 24h of the study. In our laboratory the interobserver variability for FMD is 1.02±0.6% (95% limits of agreement: -1.7-2.4%) (Savvidou MD et al. Obstet Gynecol 2000; 15: 502-7). Outside the setting of pregnancy, endothelium-independent dilatation to sublingual glyceryl trinitrate (GTN) is commonly used as a control but in the current study GTN use was avoided. However, a previous study has shown that GTN-induced dilatation is not altered as a result of pregnancy (Dorup I et al. Am J Physiol 1999; 276: H821-5).

### Analysis of Plasma Asymmetric-, Symmetric-Dimethylarginines and L-Arginine

A blood sample (5 ml) was taken into citrate tubes at the time of the vascular studies for the measurement of plasma concentrations of ADMA, SDMA and L-arginine. Thirty eight women from the control group and 40 women with bilateral uterine artery notches agreed to blood sampling. After centrifugation the plasma was stored at -70° C until assay. Endogenous amino acids and the internal standard L-homoarginine added to 0.5-ml aliquots of plasma samples (at 10 µmol/L) were solid-phase extracted on CBA cartridges (Varian, Harbor City, CA), derivatized with o-phthalaldehyde (OPA), and the OPA derivatives were separated by HPLC and monitored by fluorescence detection as described (Tsikas D et al. J Chromatogr B Biomed Sci Appl 1998; 705:174-6). OPA derivatives of L-arginine, L-homoarginine, SDMA and ADMA eluted at 8.8±0.1, 10.9±0.1, 14.8±0.2, and 16.3±0.2 min, respectively, (mean±SD, n=6). The accuracy and precision were determined within a set of six co-processed quality control samples to be closely to 100% and below 7.3%, respectively, for all amino acids.

### Definition of Clinical Outcome

Information on the course of the pregnancy, including gestational age, mode of delivery and infant birth weight was obtained for all the women studied. The clinical management of the women participating in the study was undertaken by obstetricians who were aware of the results of the uterine artery waveform recordings at 23-25 weeks, but were unaware of the results of brachial artery FMD and methylarginine measurements. Pre-eclampsia was defined according to the criteria of the International Society for the Study of Hypertension in Pregnancy (Davey et al. Am J Obstet Gynecol 1988; 158: 892-8.). Under this classification, pre-eclampsia was defined as hypertension (one diastolic blood pressure reading ≥ 110 mm Hg, or two consecutive diastolic blood pressure readings ≥90 mmHg at least four hours apart) in combination with proteinuria (≥300 mg total protein in a 24-hour urine collection or, if this was not available, 2+ proteinuria by dipstick on two consecutive occasions at least four hours apart) developing after 20 weeks of gestation in previously normotensive women. IUGR was defined as birth weight below the 5^{th} percentile for gestation and sex of the neonate (Gardosi J et al. Lancet 1992; 339: 283-7).

### Statistical Analysis

Normality of the distribution of continuous data was examined with the Shapiro-Wilk test. Logarithmic transformation was performed for non-normally-distributed data. Descriptive data are expressed as mean±SD or as median [interquartile range] for normally and non-normally distributed data. The Student's t-test was used to compare variables between the women with and without bilateral notches. Comparisons between multiple groups were performed using one-way analysis of variance followed by a post hoc test (Tukey-Kramer). Chi-square (χ²) test was used to compare categorical variables among groups. Univariate linear and multivariate regression analyses were performed where appropriate. The statistical analyses were performed using the Statistical Package for Social Sciences (Version 8).

### 2. Results

### Baseline Characteristics of the Study Participants

None of the women with normal uterine artery Doppler waveforms developed pre-eclampsia and all of them delivered infants of appropriate size. Women with bilateral notches of the uterine arteries at 23-25 weeks (n=43) were classified into three groups according to the outcome of pregnancy; those with no complications (n=19, 44.2%), those who developed IUGR (n=14, 32.6%) and those who developed PE (n=10,23.2 %), including four with IUGR.

The demographic and clinical characteristics, obtained at study entry, according to the outcome of the pregnancy are presented in Table 1. There was no statistically significant difference between the groups in baseline demographic characteristics. A greater proportion of women who subsequently developed pre-eclampsia were smokers, but the difference was not statistically significant (p=0.43). Systolic and diastolic BP were significantly higher in women who eventually developed pre-eclampsia but were nevertheless within the normal range. Women who developed pre-eclampsia had significantly higher PI of the uterine arteries and delivered smaller fetuses earlier compared to the women who did not have any complications of pregnancy.

### Maternal FMD According to the Outcome of Pregnancy

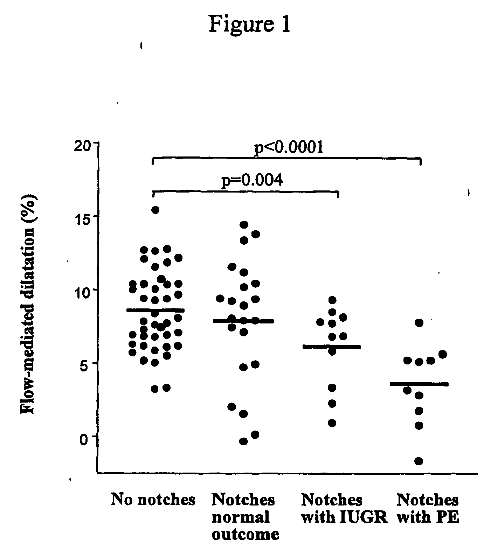

Recordings of FMD were obtained from all women and are presented in Table 2 according to the outcome of pregnancy. Women who subsequently developed pre-eclampsia, had significantly lower FMD (3.58±2.76%) than women who had no notches and normal outcome (8.59±2.76%, p<0.0001) and women who had bilateral notches but a normal outcome (8.15±4.32%, p=0.0001, Figure 1). Women whose pregnancies were complicated by IUGR also had lower FMD compared to women who had no notches and normal outcome (6.17±2.82% vs 8.59±2.76%, p=0.004). Pregnant women with normal outcome had similar FMD regardless of the absence or presence of bilateral notches (8.59±2.76% vs 8.15±4.32%, p=0.92). In a multiple regression analysis, significant predictors of FMD were baseline vessel size (p=0.002), subgroup status defined by pregnancy outcome (p<0.001) and smoking status (p<0.001). The difference in FMD among the groups remained significant even after adjustment for smoking status.

### Levels of Dimethylarginines and L-arginine

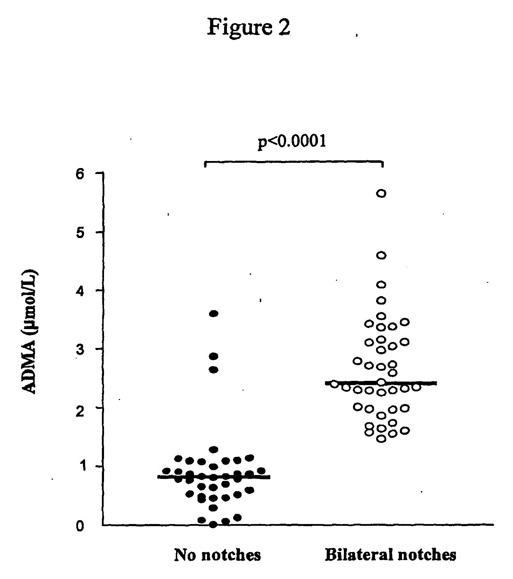

Women with bilateral notches had significantly higher levels of ADMA compared to the women with normal uterine artery Doppler waveforms (2.4 [1.97-3.14] µmol/L vs 0.81 [0.49-1.08] µmol/L respectively, p<0.0001, Figure 2). Women who subsequently developed pre-eclampsia had significantly higher levels of ADMA compared to the women who had normal pregnancies (2.7 [2.21-3.21] µmol/L vs 0.81 [0.49-1.08] µmol/L respectively, p<0.0001, Table 3). At the levels seen in women who subsequently develop pre-eclampsia, ADMA competitively inhibits the enzymatic synthesis of NO from L-arginine and attenuates endothelium-dependent relaxation. ADMA provides a mechanism for the development of pre-eclampsia and links increased placental vascular resistance with maternal hypertension resulting from systemic maternal endothelial dysfunction. The levels of symmetric dimethylarginine (SDMA; a stereoisomer of ADMA with no effect on NO synthesis) were similar in all groups, with the result that the ADMA/SDMA molar ratio was significantly higher in women with bilateral notches and pre-eclampsia (Table 3). L-arginine concentration and L-arginine/ADMA molar ratio were marginally but significantly higher in all women with bilateral notches and pre-eclampsia when compared to those without. Interestingly, despite having the lowest FMD, women who went on to develop PE had the highest rather than the lowest levels of L-arginine (Table 3).

### Correlation of ADMA with Endothelial Function

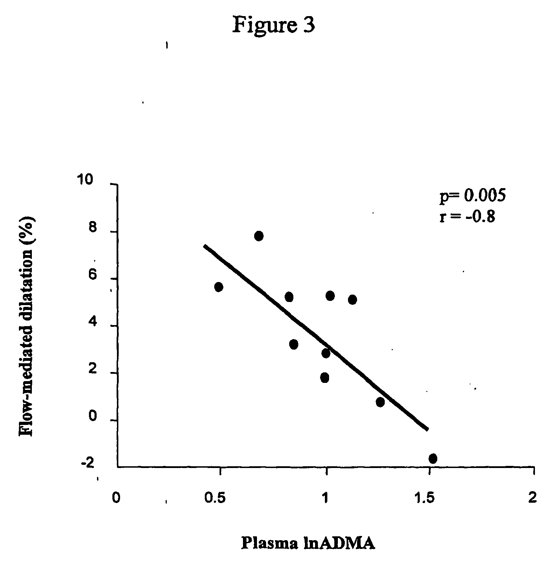

The levels of ADMA did not correlate significantly with FMD in the group of women with normal Doppler waveforms. However, there was a weak but significant inverse correlation between the plasma levels of ADMA and FMD in the group of women with bilateral uterine artery notches (r = -0.35, p=0.02). In order to investigate this relationship further, we performed univariate analyses between levels of ADMA and FMD in the three subgroups of women with bilateral notches distinguished by different pregnancy outcomes. Only in the group of pregnant women who eventually developed pre-eclampsia, there was a strong inverse correlation between ADMA and FMD (r= -0.8, p= 0.005, Figure 3).

**Table 1: Demographic, clinical maternal and neonatal characteristics in each group of women, according to the outcome of pregnancy.**

| Characteristic | No notches-Normal outcome (n=43) | Notches present-Normal outcome (n=19) | Notches present-Development of IUGR (n=14) | Notches present-Development of PE (n=10) |
|---|---|---|---|---|
| Gestational age at screening (wk) | 23 (23-25) | 24 (24-25) | 24 (24-25) | 24 (24-25) |
| | | | | |
| Maternal age (years) | 29 ± 5.3 | 27.1 ± 5.8 | 26.3 ± 6.2 | 28.2 ± 4.7 |
| | | | | |
| Smokers | 7 (16.3%) | 2 (10.5%) | 4 (28.6%) | 3 (30%) |
| | | | | |
| Nulliparity | 22 (51.2%) | 10 (52.6%) | 12 (85.7%) | 7 (70%) |
| | | | | |
| Heart rate (bpm) | 83.1 ± 10.1 | 77.5 ± 13.1 | 80.2 ± 10.6 | 79.5 ± 11.4 |
| | | | | |
| Systolic BP (mmHg) | 113.9 ± 8.9 | 114.2 ± 7.2 | 114.7 ± 9.6 | 125.6 ± 7.2* |
| | | | | |
| Diastolic BP (mmHg) | 66.7 ± 6.8 | 64.2 ± 16 | 66 ± 5.9 | 75.4 ± 9† |
| | | | | |
| Mean PI of uterine arteries | 0.81 ± 0.23 | 1.49 ± 0.6† | 1.85 ± 0.7* | 1.69 ± 0.5†. |
| | | | | |
| Gestational age at delivery (wk) | 39.4 ± 1.6 | 40 ± 1.7 | 38.6 ± 4 | 34.8 ± 3.5* |
| | | | | |
| Birth weight (gr) | 3326 ± 460 | 3207 ± 408 | 2301 ± 727* | 2074 ± 723* |

| | | | | |
|---|---|---|---|---|
| *p<0.0001, †p<0.001 All the comparisons were performed with the group of women without bilateral notches at examination. | | | | |

**Table 2: Maternal vascular characteristics in each group of women, according to the outcome of pregnancy.**

| Characteristic | No notches-Normal outcome (n=43) | Notches present-Normal outcome (n=19) | Notches present-Development of IUGR (n=14) | Notches present-Development of PE (n=10) |
|---|---|---|---|---|
| Flow-mediated dilatation, FMD (%) | 8.59 ± 2.76 | 8.15 ± 4.32 | 6.17 ± 2.82* | 3.58 ± 2.76†‡ |
| | | | | |
| Baseline vessel diameter (mm) | 3.1 ± 0.35 | 3.17 ± 0.38 | 3.3 ± 0.52 | 3.2 ± 0.4 |
| | | | | |
| Baseline blood flow (ml/min) | 182.2 (90.3-278) | 181.5 (82.5-210.6) | 128.6 (86-319) | 194.4 (100-367) |
| | | | | |
| Reactive hyperemia (%) | 473 (245-805) | 614.5 (359-925) | 605.5 (215-926) | 381.3 (163-940) |

| | | | | |
|---|---|---|---|---|
| *p=0.004, comparison with women without notches and normal outcome. †p<0.0001, comparison with women without notches and normal outcome. ‡p<0.0001, comparison with women with notches and normal outcome. | | | | |

**Table 3: Levels of ADMA, SDMA and L-arginine in each group of women, according to the outcome of pregnancy.**

| Characteristic | No notches-Normal outcome (n=38) | Notches present-Normal outcome (n=16) | Notches present-Development of IUGR (n=14) | Notches present-Development of PE (n=10) |
|---|---|---|---|---|
| Plasma ADMA | 0.81 | 1.99* | 3.04* | 2.78 |
| (µmol/L) | (0.49-1.08) | (1.69-2.38) | (2.39-3.54) | (2.21-3.21) |
| | | | | |
| Plasma SDMA | 0.61 | 0.51 | 0.54 | 0.51 |
| (µmol/L) | (0.43-0.76) | (0.18-0.69) | (0.34-0.7) | (0.27-0.77) |
| | | | | |
| Plasma L-arginine | 21.9 | 23.5 | 28.3† | 31.1* |
| (µmol/L) | (19.4-25.8) | (22.2-27.6) | (23.6-33.7) | (24.6-35) |
| | | | | |
| ADMA/SDMA | 1.27 | 4.6* | 5.65* | 6.8* |
| molar ratio | (0.97-1.68) | (3.1-13.5) | (3.97-8.87) | (3.92-8.72) |
| | | | | |
| L-arginine/ADMA | 31.1 | 11.09* | 8.93* | 11.27* |
| molar ratio | (21.4-39.7) | (8.85-15) | (7.36-14.17) | (8.74-15.17) |

| | | | | |
|---|---|---|---|---|
| *p<0.0001, †p<0.005 All the comparisons were performed with the group of women without bilateral notches at examination. | | | | |

## Claims

1. A method of identifying whether or not a pregnant woman is at risk of developing pre-eclampsia or whether or not her fetus is at risk of developing intrauterine growth restriction (IUGR), which method comprises:
a) measuring asymmetric dimethylarginine (ADMA) in a fluid sample taken from a pregnant woman at a stage of pregnancy from 4 to 25 weeks gestation; and
b) determining whether or not the ADMA is greater than 2.0µmol/L in the fluid sample, thereby determining whether or not the woman is at risk of developing pre-eclampsia or her fetus is at risk of developing IUGR.

2. A method according to claim 1, wherein the pregnant woman is at a stage of pregnancy from 10 to 25 weeks gestation.

3. A method according to claim 2, wherein the woman is at a stage of pregnancy from 15 to 25 weeks gestation.

4. A method according to any one of the preceding claims, wherein determining whether or not the woman is at risk of developing pre-eclampsia or determining whether or not her fetus is at risk of developing IUGR comprises determining whether or not the woman's ADMA level is at least 3 times the normal pregnancy level.

5. A method according to any one of the preceding claims, wherein determining whether or not the woman is at risk of developing pre-eclampsia or determining whether or not her fetus is at risk of developing IUGR comprises determining whether or not the woman has an increase in the ADMA/symmetric dimethylarginine (ADMA/SDMA) ratio that is greater than the normal pregnancy ratio.

6. A method according to claim 5, comprising determining whether or not the ADMA/SDMA ratio is at least 5 times more than the normal pregnancy ratio.

7. A method according to any one of the preceding claims, wherein the pregnant woman is suspected of being at risk of developing pre-eclampsia or her fetus is suspected of being at risk of developing IUGR.

8. A method according to claim 7, wherein the woman is a smoker.

9. A method according to any one of the preceding claims, further comprising carrying out Doppler waveform analysis of the uterine arteries and/or flow-mediated dilatation of the brachial artery in the woman.

## Patentansprüche

1. Verfahren zum Erkennen, ob eine schwangere Frau gefährdet ist, eine Präeklampsie zu entwickeln oder nicht, oder ob ihr Fötus gefährdet ist, intrauterinen Kleinwuchs (IUGR) zu entwickeln oder nicht, worin das Verfahren umfasst:
a) Messen von asymetrischem Dimethylarginin (ADMA) in einer Fluidprobe, welche einer schwangeren Frau in einem Schwangerschaftsstadium von der 4. bis zur 25. Schwangerschaftswoche entnommen wird; und
b) Bestimmen, ob der ADMA-Wert in der Fluidprobe größer ist als 2,0 µmol/l, wobei bestimmt wird, ob die Frau gefährdet ist, eine Präeklampsie zu entwickeln oder nicht, oder ihr Fötus gefährdet ist, IUGR zu entwickeln oder nicht.

2. Verfahren nach Anspruch 1, worin sich die schwangere Frau in einem Schwangerschaftsstadium von der 10. bis zur 25. Schwangerschaftswoche befindet.

3. Verfahren nach Anspruch 2, worin sich die Frau in einem Schwangerschaftsstadium von der 15. bis zur 25. Schwangerschaftswoche befindet.

4. Verfahren nach einem der vorhergehenden Ansprüche, worin die Bestimmung, ob die Frau gefährdet ist, eine Präeklampsie zu entwickeln oder nicht, oder die Bestimmung, ob ihr Fötus gefährdet ist, IUGR zu entwickeln oder nicht, die Bestimmung umfasst, ob der ADMA-Spiegel der Frau mindestens das 3-fache des normalen Schwangerschaftsspiegels beträgt oder nicht.

5. Verfahren nach einem der vorhergehenden Ansprüche, worin die Bestimmung, ob die Frau gefährdet ist, eine Präeklampsie zu entwickeln oder nicht, oder die Bestimmung, ob ihr Fötus gefährdet ist, IUGR zu entwickeln oder nicht, die Bestimmung umfasst, ob die Frau einen Anstieg im Verhältnis ADMA/symmetrisches Dimethylarginin (ADMA/SDMA) aufweist oder nicht aufweist, welches größer ist als das normale Schwangerschaftsverhältnis.

6. Verfahren nach Anspruch 5, umfassend die Bestimmung, ob das Verhältnis ADMA/SDMA mindestens 5-mal mehr beträgt als das normale Schwangerschaftsverhältnis oder nicht.

7. Verfahren nach einem der vorhergehenden Ansprüche, worin die schwangere Frau im Verdacht steht, bezüglich der Entwicklung einer Präeklampsie gefährdet zu sein oder ihr Fötus im Verdacht steht, bezüglich der Entwicklung von IUGR gefährdet zu sein.

8. Verfahren nach Anspruch 7, worin die Frau eine Raucherin ist.

9. Verfahren nach einem der vorhergehenden Ansprüche, weiter umfassend Durchführen einer Doppler-Wellenform-Analyse der Uterusarterien und/oder einer flussabhängigen Dilatation der Oberarmarterie der Frau.

## Revendications

1. Procédé pour identifier si une femme enceinte risque de développer un éclampsisme ou si le foetus risque de développer un retard de croissance intra-utérin (IUGR), lequel procédé comprend les étapes consistant à :
a) mesurer la diméthylarginine asymétrique (ADMA) dans un échantillon de fluide prélevé sur une femme enceinte à un stade de grossesse de 4 à 25 semaines de gestation ; et
b) déterminer si l'ADMA est supérieure à 2,0 µmol/L dans l'échantillon de fluide, déterminant ainsi si la femme risque de développer un éclampsisme ou si le foetus risque de développer un IUGR.

2. Procédé selon la revendication 1, dans lequel la femme enceinte est à un stade de grossesse de 10 à 25 semaines de gestation.

3. Procédé selon la revendication 2, dans lequel la femme est à un stade de grossesse de 15 à 25 semaines de gestation.

4. Procédé selon l'une quelconque des revendications précédentes, dans lequel le fait de déterminer si la femme risque de développer un éclampsisme ou le fait de déterminer si le foetus risque de développer un IUGR comprend le fait de déterminer si le taux d'ADMA de la femme correspond à au moins 3 fois le taux normal de grossesse.

5. Procédé selon l'une quelconque des revendications précédentes, dans lequel le fait de déterminer si la femme risque de développer un éclampsisme ou le fait de déterminer si le foetus risque de développer un IUGR comprend le fait de déterminer si le rapport ADMA/diméthylarginine symétrique (ADMA/SDMA) de la femme présente une augmentation qui est supérieure au rapport normal de grossesse.

6. Procédé selon la revendication 5, comprenant le fait de déterminer si le rapport ADMA/SDMA est au moins 5 fois supérieur au rapport normal de grossesse.

7. Procédé selon l'une quelconque des revendications précédentes, dans lequel la femme enceinte est suspectée d'avoir un risque de développer un éclampsisme ou le foetus est suspecté d'avoir un risque de développer un IUGR.

8. Procédé selon la revendication 7, dans lequel la femme est fumeuse.

9. Procédé selon l'une quelconque des revendications précédentes, comprenant en outre la réalisation d'une analyse de forme d'onde Doppler des artères utérines et/ou une dilatation dépendante du flux sanguin de l'artère brachiale de la femme.
